# EUROPEAN PATENT APPLICATION

(11) **EP 1 550 476 A1**
(43) Date of publication of application: **06.07.2005**
(21) Application number: 04025250.4
(22) Date of filing: 23.10.2004
(51) Int. Cl.: A61M 5/32

(54) **Syringe with retractable needle**

(30) Priority: 30.12.2003 US 747053
(71) Applicant: Life-Shield Products, Inc., Taipei (TW)
(72) Inventor: Shih, Chao-Hua, Wugu Shiang Taipei (TW)
(74) Representative: Meyer, Ludgerus A.

(57) **Abstract**

A retractable safe syringe, including a needle set (40), a cylinder (50) and a plunger (60). The cylinder is formed as a needle-set socket on its upper portion. The needle set has a first engaging portion (43) on the bottom, and is connected to the needle-set socket with a predetermined force. The plunger has a piston (61) on its front portion, and a second engaging portion (64) is provided on the front end of the position. The piston is made of elastic material, has a hollow space therein, and is in a shape easy to be compressed and restored. The piston can be pushed upwards in the cylinder until the top of the piston contacts the top wall in injecting space of the cylinder at the situation that first engaging portion is not connected with the second engaging portion. When a force larger than a value is exerted, the piston is pressed to deform to an extent to make the second engaging portion to protrude upwards far out of the top of the piston and to connect with the first engaging portion, whereof the needle set can be released from the needle-set socket and retracted into the cylinder.

## Description

### BACKGROUND OF THE INVENTION

### 1. Field of the invention

The present invention is related to a safe syringe with a needle retractable into a cylinder, especially to a safe syringe having an elastic piston which is compressible and retractable with a force, and applicable to various retractable safe syringes.

### 2. Description of the Prior Art

Injectors of syringes for subcutaneous injection used presently have been widely applied on the medical field, generally a syringe is discarded after being used once, this is convenient and sanitary and can avoid infection of deceases through a needle set; however, the syringe discarded is exposed to the outside, it may stab and hurt a medical staff to make a wound or infect a decease. Wasted syringes will also stab and hurt people with their sharp needles if they are randomly discarded without well treatment; environmental personnel who treat the medical wastes may also be the victims of the wasted needles. Therefore, retractable type safe syringes have been developed to retract the needle sets after use; the needle sets can be hidden in cylinders of syringes to reduce the volume of the syringes.

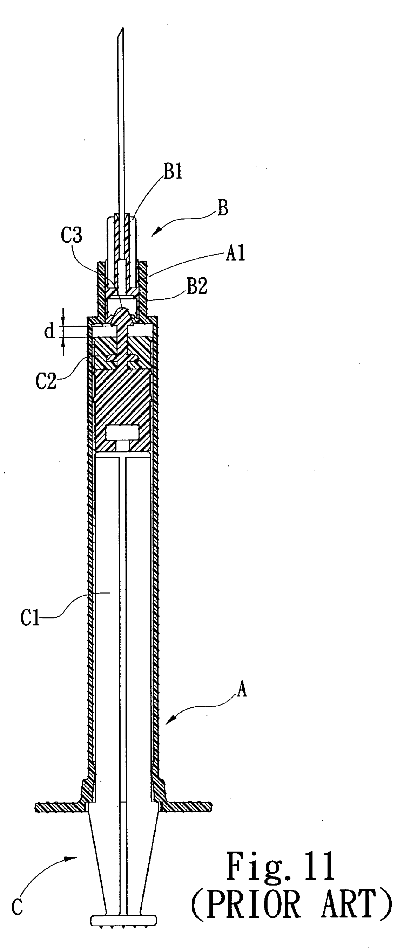

A conventional retractable type safe syringe is shown in Fig. 11, wherein a cylinder A is formed on the front end thereof a socket A1, the socket A1 can be used for fixing a needle set B which includes a needle seat B1 having on its bottom a first engaging portion B2. A plunger C having a pusher rod C1 and a piston C2 can be pushed in from the rear of the cylinder A; the piston C2 is provided with a second engaging portion C3 on its top, the first engaging portion B2 and the second engaging portion C3 are left therebetween with a gap d in advance in order that inadvertent connecting of them with each other can be avoided; a force is required to push the pusher rod C1 forwards in the last stage of injection of medicine, so that the first engaging portion B2 and the second engaging portion C3 can be connected with each other, hence when the pusher rod C1 is pulled backwards, the needle set B with a needle can thus be pulled into the cylinder A, and the syringe can be discarded safely. There are safe syringes provided with spring retracting mechanisms and activating devices for automatically retracting needles in the markets; when a force is exerted on an activating device after injection, a needle set will immediately be retracted into a cylinder of a syringe by a spring retracting mechanism; however, in order to avoid inadvertent connecting of a pusher rod with the needle set before injection, the front end of the pusher rod of the syringe and the needle set still are left therebetween with a gap, this makes an amount of error in drawing medicine. In view of these, the above stated syringe still has the following defects:
1. In most of the conventional syringes, a gap (or dead space) is existed between the top surface of the plunger and the engaging portion of the needle set when drawing medicine into cylinder to avoid inadvertent connecting of them with each other and the gap often makes inaccuracy of the required amount of medicine.
2. In the last stage of medicine injection for a manual retractable safe syringe, a quite amount of force is required to apply on the plunger to completely push out the medicine and to overcome impedance against engaging and connecting the plunger with the needle. However, the force may make hurt of a patient in medicine injection.

In view of these, the inventor develops the present invention to solve the problems after hard study.

### SUMMARY OF THE INVENTION

The primary object of the present invention is to provide a retractable safe syringe having an elastic piston which is compressible and retractable with a force so that the syringe can help or semi automatically render a needle set to retract into a cylinder, and is convenient for operation.

The secondary object of the present invention is to provide a retractable safe syringe which allows its elastic piston to be pushed to contact a top end wall of the cylinder at the situation that the needle set and the plunger is not engaged, so that a user can accurately drawing medicine from a mark zero on the cylinder, thereby good dosage control can be obtained, and that medicine can be injected completely, thereby avoiding hurst on the patient during injection. The engagement of the needle set and the plunger can be operated after the syringe is taken out of the body of the patient.

To achieve the above objects, the retractable safe syringe of the present invention is comprised of a needle set, a cylinder and a plunger, wherein the needle set has a first engaging portion on the bottom thereof; the cylinder is formed as a needle-set socket on its upper portion and to have its lower portion a space for injection; the needle set is connected to the needle-set socket with a predetermined connecting force, the plunger has a piston which is made of elastic material, has a hollow space therein, and is in a shape easy to be compressed and restored;and a second engaging portion is disposed on the front end of the piston.

Further, the present invention make arrangement that the piston can be pushed upwards in the injection space of the cylinder till the top surface of the piston contacts the top end wall of the cylinder at the situation that the first engaging portion is not engaged with the second engaging portion. When the force of pushing the piston is continuously applied, the piston is deformed to certain extent to render the second engaging portion to protrude upwards far out of the top surface of the piston, and connect with the first engaging portion.

The present invention provides several kinds of embodiments of various shapes and structures of pistons. The pistons can help the user to overcome the predetermined connecting force of the needle set with the cylinder by a restoring force after compressing. The present invention also provides several kinds of embodiments of the way that the second engaging portion is disposed on the front end of the piston.

The present invention will be apparent after reading the detailed description of the preferred embodiments thereof in reference to the accompanying drawings.

### BRIEF DESCRIPTION OF THE DRAWINGS

Fig. 1 is an analytic perspective view showing an embodiment of a retractable safe syringe of the present invention;
Fig. 2 is a schematic sectional view of the embodiment as shown in Fig. 1.
Fig. 2A is a partially enlarged schematic sectional view of Fig. 2;
Fig. 2B is a partially enlarged schematic sectional view of Fig. 2, showing the situation of the top surface of the piston contacted with the top end wall in the injecting space of the cylinder before the piston is deformed;
Fig. 3 is a schematic sectional view of the embodiment of Fig. 1, showing the situation that the first engaging portion is connected with the second engaging portion;
Fig. 3A is a partially enlarged schematic sectional view of Fig. 3;

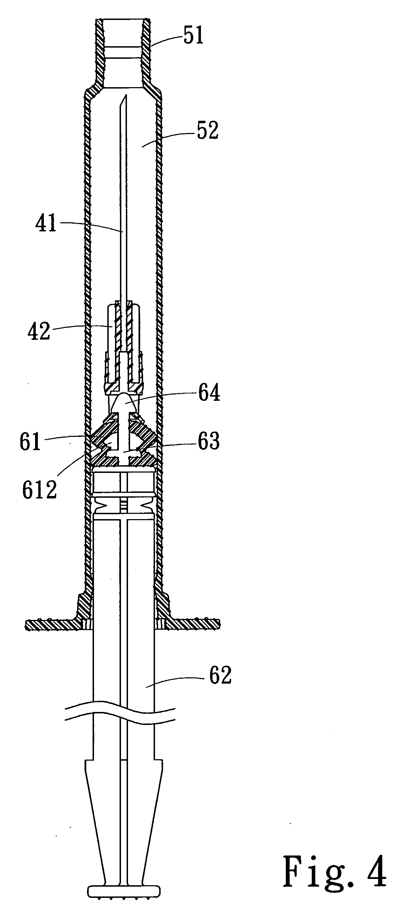

Fig. 4 is a schematic sectional view of the embodiment of Fig. 1, showing the situation that the needle set is drawn into the cylinder of the syringe;
Fig. 5 is a schematic sectional view of another embodiment of the present invention, showing a piston formed by stacking a plurality of round disks;
Fig. 5A is a partially enlarged schematic sectional view of Fig. 5;
Fig. 6 is a schematic sectional view of a further embodiment of the present invention, showing a piston with its middle neck reduced;
Fig. 6A is a partially enlarged schematic sectional view of Fig. 6;
Fig. 7 is a schematic sectional view of a further embodiment of the retractable safe syringe of the present invention, showing a piston with a surface thread;
Fig. 7A is a partially enlarged schematic sectional view of Fig. 7;
Fig. 8 is a schematic sectional view of an embodiment of the retractable safe syringe of the present invention, showing a hook portion is disposed at a position partially protruding out of the top surface of a piston;
Fig. 8A is a partially enlarged schematic sectional view of Fig. 8;
Fig. 9 is a schematic sectional view of another embodiment of another retractable safe syringe of the present invention, showing the hook portion is disposed at a position completely hidden inside a piston;
Fig. 9A is a partially enlarged schematic sectional view of Fig. 9;
Fig. 10 is a schematic sectional view of an embodiment of the retractable safe syringe of the present invention applied to standard needle which is changeable.
Fig. 10A is a partially enlarged schematic sectional view of Fig. 10; and
Fig. 11 is a schematic sectional view of a conventional retractable type safe syringe.

### DETAILED DESCRIPTION OF THE PREFERRED EMBODIMENT

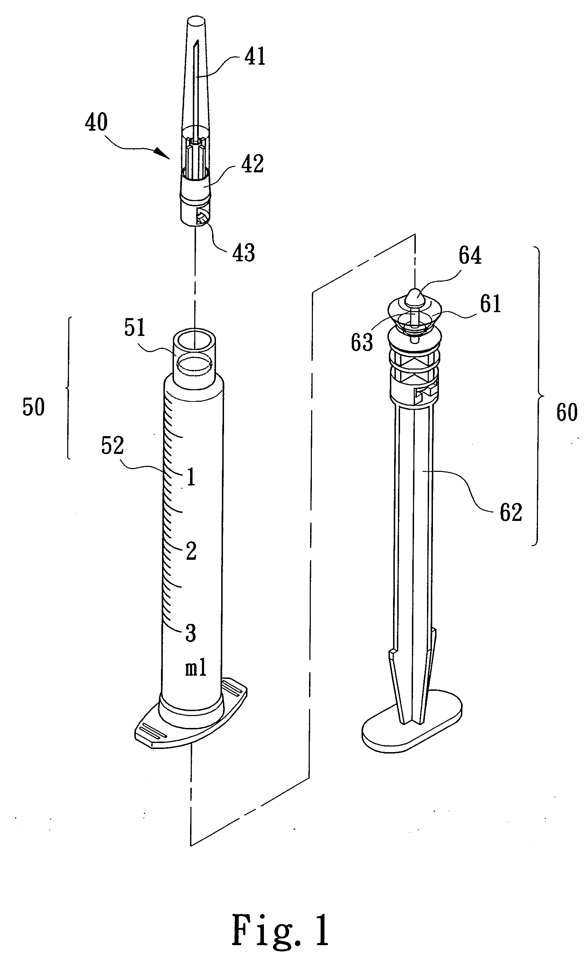

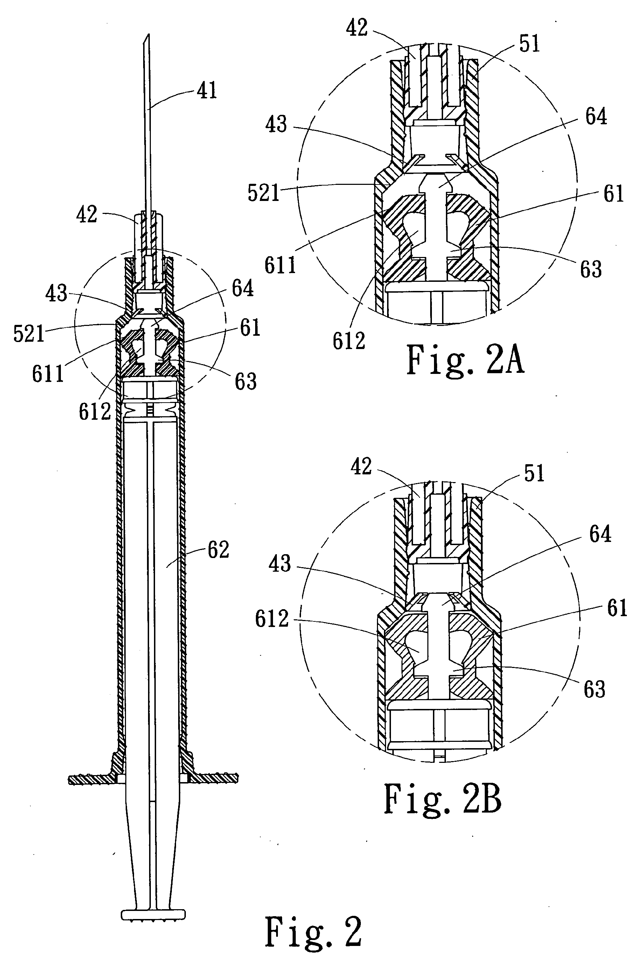

Referring to Figs. 1, 2 and 2A, the retractable safe syringe of the present invention comprises: a needle set 40, a cylinder 50 and a plunger 60, wherein the needle set 40 at least includes a needle 41 and a needle seat 42 for fixing the needle 41, the needle seat 42 has a first engaging portion 43 on the bottom thereof, the first engaging portion 43 formed by at least a detent, which is only an embodiment, the lower end of the first engaging portion 43 is fixed on an inner bottom wall of the needle seat 42, while the upper end is extended upwards and forms a cone with a reduced upper opening, the first engaging portion 43 is centrally hollow to be communicated with the needle seat 42 and the needle 41 to form a passage for medicine.

The plunger 60 can be moved from below the cylinder 50 into the injection space 52 of the cylinder 50; the plunger 60 is provided on its lower portion with a pusher rod 62, and is provided on its front end centrally with a piston shaft 63, an elastic piston 61 is flexibly sleeved on the piston shaft 63; the piston 61 is made of elastic material and has therein a hollow space 612, and is in a shape that is easy to be compressed and restored; the piston shaft 63 has on its top a second engaging portion 64 which is a conical hook protruding upwards out of the top surface 611 of the piston 61 in this embodiment, the bottom of the hook engaging portion 64 contacts the top surface of the elastic piston 61.The first engaging portion 43 and the second engaging portion 64 can engage with each other, and construct a engaging mechanism.

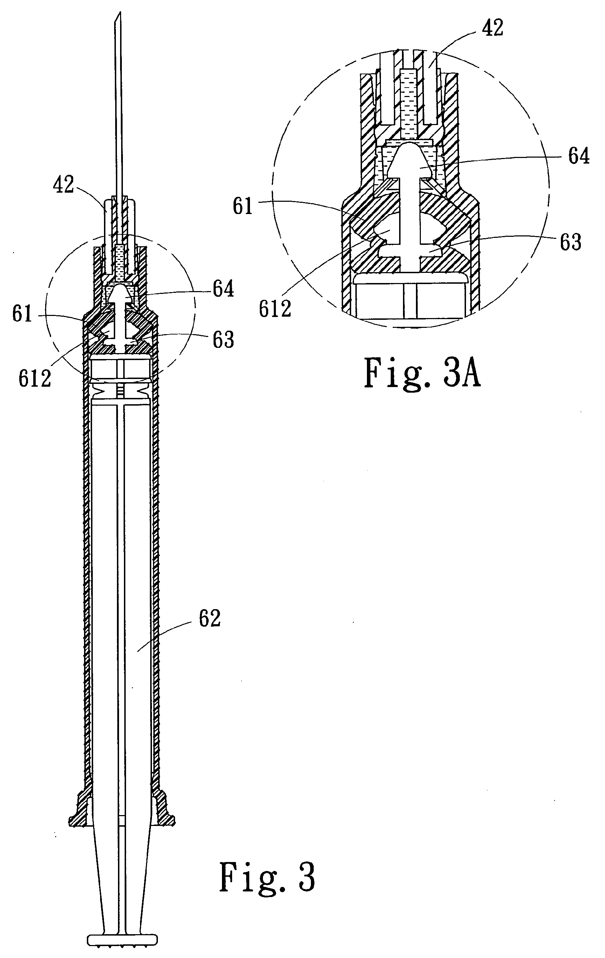

As shown in Figs. 1, 2 and 2A, the piston 61 in this embodiment is composed of two conical elastic members stacking one over the other, the elastic members have on their round peripheries closely clung to the inner wall of the cylinder 50; when a doctor or a nurse is going to draw medicine, the top surface 611 of the piston 61 can be pushed to contact a top end wall 521 in the injecting space 52 of the cylinder 50; at this time, the first engaging portion 43 is not yet connected with the second engaging portion 64 (referring to Fig. 2B), so that a user can draw medicine starting from a mark zero (beginning marking) on the cylinder. And during injecting the medicine, the pusher rod 62 is pushed forward until the top surface 611 of the piston is contacted with the top end wall 521 in the injecting space 52 so that all the medicine is pushed out of the cylinder 50; at this time, the first engaging portion 43 is not yet connected with the second engaging portion 64 either. After completion of injection in order to withdraw the needle set 40 into the cylinder 50, the needle set 40 can be removed from the body of a patient, and the pusher rod 62 is pushed forwards by a force to press and deform the piston 61; meantime, the first engaging portion 43 is connected with the second engaging portion 64 such as are shown in Figs. 3 and 3A. When the force is released and the doctor or nurse is in a proceeding to withdraw the needle set, the restoring force of the piston 61 can help to overcome the connection force of the needle set 40 with a needle-set socket 51, and help to pull the needle set 40 into the cylinder 50; at this time, it needs only that the user slightly pulls rearwards the pusher rod 62, the needle set 40 thus can be semi automatically pulled back to into the cylinder 50 so that the needle set 40 will not be exposed out of the cylinder 50 and can be safely discarded.

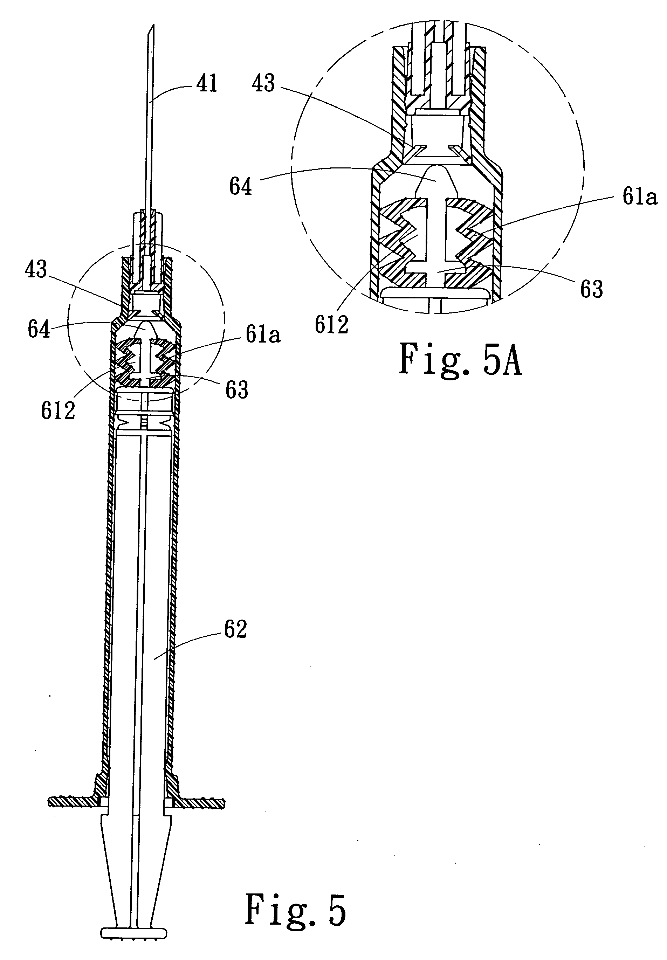

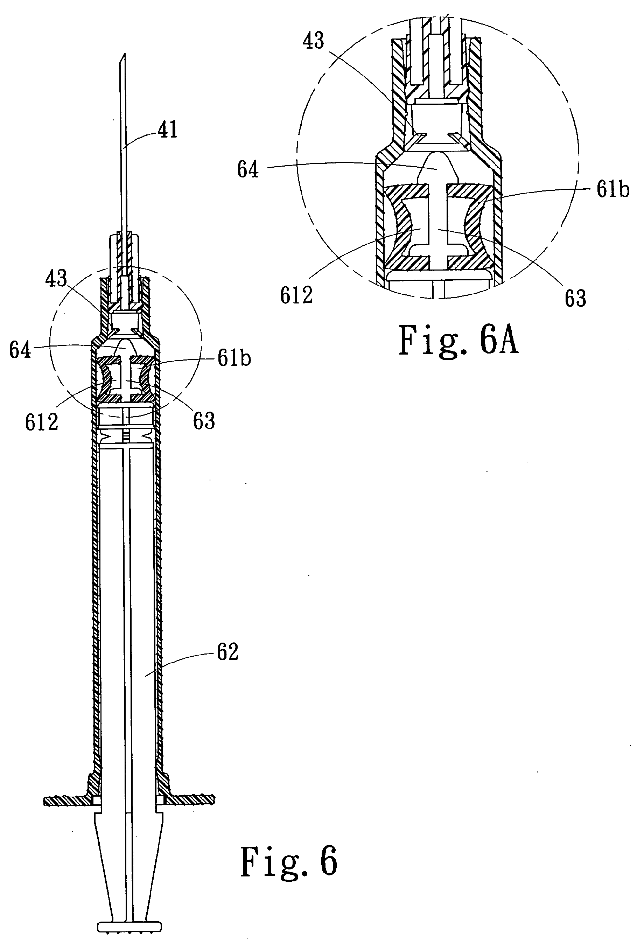

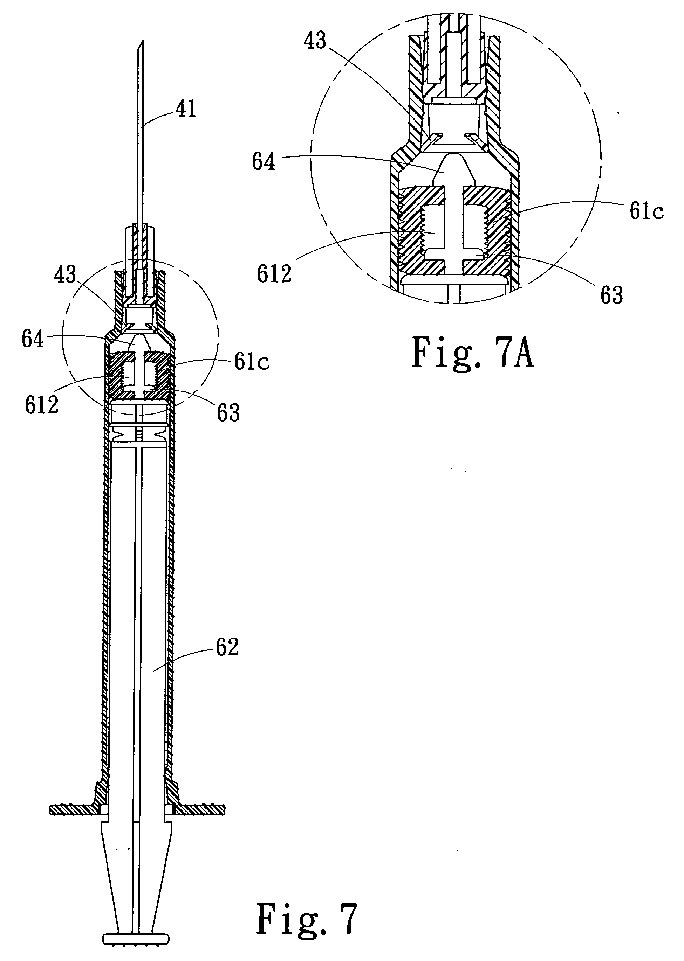

In addition to that shown in Fig. 1, the conformation of the piston can be designed that the piston 61a has at least two round-disk shaped elastic members stacking one over the other, such as are shown in Figs. 5 and 5A.It can be also designed that the piston 61b be with a reduced middle neck portion, such as are shown in Figs. 6 and 6A. It can be further designed that the piston 61c be a column with a thread on its surface, such as are shown in Figs. 7 and 7A.

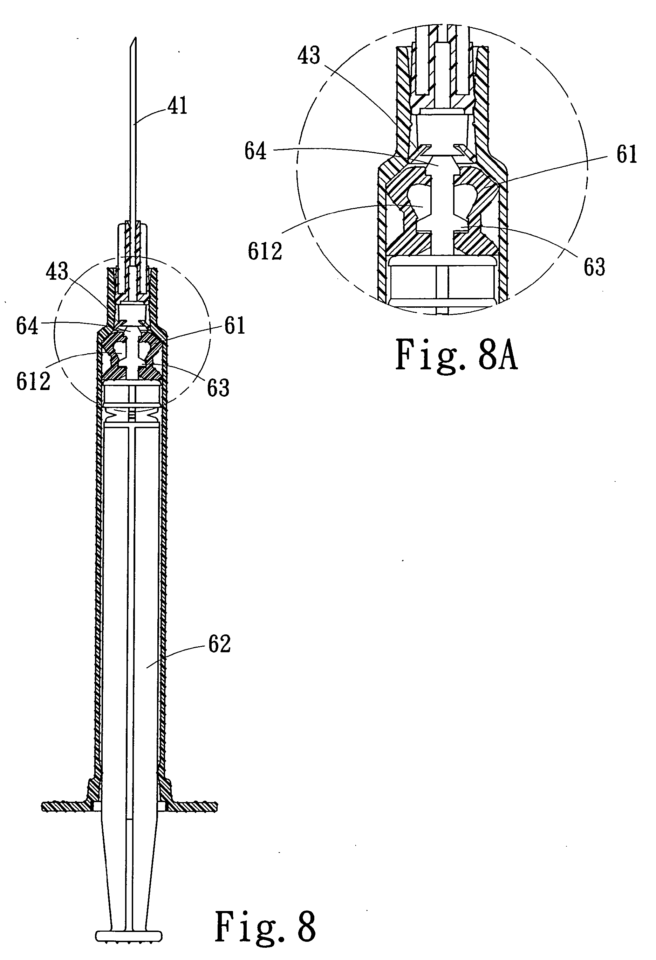

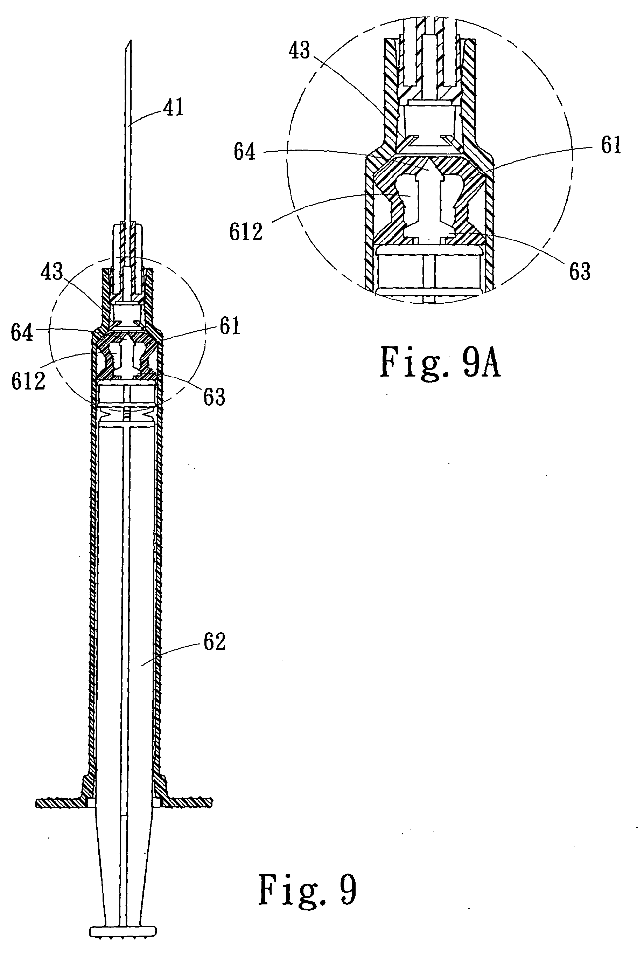

Further, in addition to the design that the hook or second engaging portion 64 is at a position protruding out of the top surface 611 of the piston 61, the hook 64 can also be disposed at different position relative to the top surface of the piston in pursuance of the requirement of production or using. Such as are shown in Fig. 8, the front portion of the hook 64 protrudes out of the top surface 611 of the piston 61, while the remaining portions of the hook 64 are positioned in the hollow space 612 of the piston 61;Further, as shown in Fig. 9, the hook 64 could be totally disposed within the piston 61 (i.e., they are enveloped by the piston 61). When a force is exerted to press and deform the piston 61, the hook 64 can be extended to protrude upwards far out of the top surface 611 of the piston 61 to connect with the first engaging portion 43. Therefore, no matter the position of the hook 64 is completely exposed to the outside of the piston 61 (as shown in Fig. 2), partially exposed to the outside of the piston 61 (as shown in Fig. 8A) or completely enveloped in the piston 61 (as shown in Fig. 9A), once a force is exerted to press and deform the piston 61, the hook 64 can be extended to protrude upwards far out of the top surface 611 of the piston 61 to connect with the first engaging portion 43 without impediment.

When in implementation, the first engaging portion and the second engaging portion of the present invention can also be other shapes which can connect with each other. All modifications or changes with the same technical features shall also be deemed as within the scope of this invention.

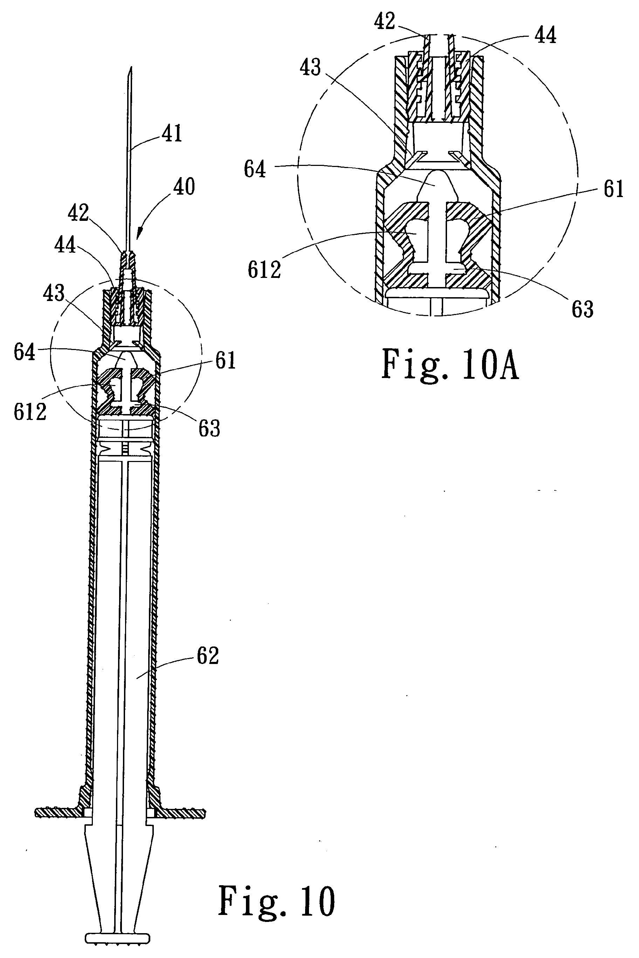

Again referring to Fig. 10, the needle set 40 of the present invention further can include an adapter 44. The adapter 44 is used to connect onto the cylinder 50, wherein the needle 41 and the needle seat 42 are detachably connected in the adapter 44, while a first engaging portion 43 is formed on the bottom of the adapter 44. The needle 41 can be changed between the actions of drawing liquid medicine and injection by the arrangement that the needle 41 and the needle seat 42 be rotatively connected (lure lock) or direct slipped and engaged (lure slip); Fig. 10 shows an example of rotatively connecting of the needle with the needle seat. Other retractable syringes with different shapes and connecting modes of the needle with the needle seat could also use the technic means of the present invention mentioned above.

The structure disclosed by the present invention can be used in various retractable safe syringes of the medical field, and thus, the present invention has the following advantages:
1. The resotoring force of the piston according to the present invention after compressing can help to overcome the connection force of the needle set with the needle-set socket, so that a user of the syringe can semi automatically retract the needle set into the cylinder and save energy.
2. The retractable safe syringe of the present invention may be taken out of the body of a patient after that the injection medicine is all pushed out of the cylinder. Then to force the plunger to connect with the needle set outside the body of patients, and thus can avoid causing hurt or discomfort to the patients.
3. A considerable amount of force shall be exerted to connect the needle set with the plunger, and it is easier to avoid undue connecting before use or during transportation according to present invention.
4. Before injection by the retractable safe syringe of the present invention, the top surface of the piston can be pushed to the end to contact a top end wall in the injecting space of the cylinder, so that a user can completely exhaust air out of the cylinder, and accurately drawing medicine from a mark zero on the cylinder, thereby good dosage control can be obtained.

As stated in the above disclosed, the present invention can surely attain its expected objects to provide a retractable safe syringe which is of simple structure, convenient for use and industrially valuable.

## Claims

1. A retractable safe syringe comprising:
a needle set, including a needle and a needle seat for fixing said needle, said needle set has a first engaging portion on its bottom;
a cylinder, being formed as a needle-set socket on its upper poetion, and a space for injection in its lower portion, said needle set is connected to said needle-set socket with a predetermined connecting force;
a plunger, adapted for moving from below said cylinder into said injection space of said cylinder, said plunger includes a piston on its front portion wherein a second engaging portion is provided on the front end of the piston for engaging with said first engaging portion, said piston is made of elastic material, has a hollow space therein, and is in a shape that is easy to be compressed and restored;
wherein said piston is allowed to be pushed upwards in said injection space of said cylinder until a top surface of said piston contacts a top end wall in said injecting space at the situation that said first engaging portion is not connected with said second engaging portion; and when the needle set is moved out of the body of patients and a force larger than a predetermined value is exerted, said piston is pressed to deform to a predetermined extent to render said second engaging portion to protrude upwards far out of said top surface of said piston, and then said first engaging portion is allowed to connect with said second engaging portion to render said needle set to get rid of said needle-set socket and to be retracted into said cylinder.

2. The retractable safe syringe as claimed in claim 1, wherein said second engaging portion of said plunger is a hook protruding upwards out of said top surface of said piston, and the bottom of said hook contacts said surface of said piston.

3. The retractable safe syringe as claimed in claim 1, wherein, said second engaging portion of said plunger is a hook of which a front portion protrudes upwards out of said top surface of said piston, while the remaining portions of said hook are received in said piston.

4. The retractable safe syringe as claimed in claim 1, wherein said second engaging portion of said plunger is a hook which is hidden and received in said piston, and when a force larger than a predetermined value is exerted to press and deform said piston, said hook is extended to protrude upwards far out of said top surface of said piston to connect with said first engaging portion.

5. The retractable safe syringe as claimed in claim 1, wherein said piston is composed of two conical elastic members stacking one over the other.

6. The retractable safe syringe as claimed in claim 1, wherein said piston is composed at least of two round-disk shaped elastic members stacking one over the other.

7. The retractable safe syringe as claimed in claim 1, wherein said piston is in a shape of a column with a reduced middle neck portion.

8. The retractable safe syringe as claimed in claim 1, wherein said piston is in a shape of a column with a thread on its surface.

9. The retractable safe syringe as claimed in claim 1, wherein said needle set further includes an adapter, said needle and said needle seat are detachably connected in said adapter, while said first engaging portion is formed on the bottom of said adapter.
